(19) **Europäisches Patentamt**
**European Patent Office**
**Office européen des brevets**

(11) **EP 1 953 548 A2**

(12) **EUROPEAN PATENT APPLICATION**

(43) Date of publication:
**06.08.2008 Bulletin 2008/32**

(51) Int Cl.:
***G01N 33/50*** (2006.01) ***A61Q 17/04*** (2006.01)

(21) Application number: **08250390.5**

(22) Date of filing: **01.02.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **02.02.2007 US 670611**

(71) Applicant: **Johnson & Johnson Consumer Companies, Inc.**
**Skillman, NJ 53008 (US)**

(72) Inventors:
• **Garay, Michelle**
**Pittstown**
**NJ 08867 (US)**
• **Marrs, Christopher**
**Rancho Palos Verdes**
**CA 90275 (US)**
• **Southall, Michael**
**Lawrenceville**
**NJ 08648 (US)**

(74) Representative: **Kirsch, Susan Edith et al**
**Carpmaels & Ransford**
**43-45 Bloomsbury Square**
**London WC1A 2RA (GB)**

(54) **Assessment and mitigation of oxidative stress in skin**

(57) A method of assessing oxidative stress in a mammal is provided. The method comprises: a) exposing skin cells of said mammal to an oxidizable moiety; b) exposing said skin cells to an external aggression; and c) assessing a reaction product of said oxidizable moiety; wherein prior to said assessing step (c), said skin cells are non-invasively removed from said mammal such that said removed skin cells are viable. A personal care composition comprises a sunscreen, wherein said composition has an oxidation protection factor of at least about 40%.

EP 1 953 548 A2

**Description**

## FIELD OF THE INVENTION

The present invention relates to method of assessing oxidative stress in skin and, in particular, to a method of assessing the ability of a topical composition to mitigate oxidative stress from an external aggression.

## BACKGROUND OF THE INVENTION

Life forms generally strive to maintain a chemical environment within their cells that is beneficial to and supports various critical biochemical processes. External factors can create biochemical disturbances and can cause toxic effects through the production of peroxides and free radicals that damage cell components, such as lipids and DNA. In particular, external factors may cause so called "oxidative stress," a disturbance in the normal redox state within the cell. A particularly destructive aspect of oxidative stress is the production of reactive oxygen species ("ROS"), which include free radicals and peroxides. Some of the less reactive of these species can be converted by oxidoreduction reactions with transition metals into more aggressive radical species that can cause extensive cellular damage. Most of these oxygen-derived species are produced at a low level by normal aerobic metabolism and the damage they cause to cells is constantly repaired. However, under the severe levels of oxidative stress that cause necrosis, the damage causes ATP depletion, preventing controlled apoptotic death and causing the cell to simply fall apart.

Oxidative stress has been the subject of much research, and various extrinsic factors have been postulated as having an influence on the level of oxidative stress in cells. For example, it is believed that prolonged exposure to UV radiation can trigger or accentuate the formation of damaging ROS.

Clearly it is desirable to find ways to detect and to reduce the level of oxidative stress within the body. However, conventional methods for assessing the effects of oxidative stress on skin involve invasive methods for harvesting skin cells (such as via skin biopsy), costly clinical studies, or invasive methods of collecting viable cells. Conventional *in vitro* methods attempt to assess oxidative stress by simulating the effects of external aggressions on "cultured" cells obtained in various manners, and thus measure effects that do not capture a complete biological response.

Furthermore, conventional methods typically attempt to quantify the content of an antioxidant in the removed cells or measurements via the use of expensive and complex instrumentation, such as high performance liquid chromatography. These instruments are cumbersome, not easily transported, expensive, require extensive training to use, and are have poor sensitivity of detection.

Thus, applicants have observed that the teachings of the prior art provide neither (a) topical compositions that provide a high level of protection from oxidative stress nor (b) a simple, inexpensive, reliable, non-invasive, and/or more complete means of assessing the ability of biological systems to respond to oxidative stress. Accordingly, it would be desirable to overcome one or more of the above-mentioned drawbacks.

## SUMMARY OF THE INVENTION

In one aspect of the invention, a method of assessing oxidative stress in a mammal is provided. The method comprises: a) exposing skin cells of said mammal to an oxidizable moiety; b) exposing said skin cells to an external aggression; and c) assessing a reaction product of said oxidizable moiety; wherein prior to said assessing step (c), said skin cells are non-invasively removed from said mammal such that said removed skin cells are viable.

In another aspect of the invention, a personal care composition comprises a sunscreen, wherein said composition has an oxidation protection factor of at least about 40%.

Other features and advantages of the present invention will be apparent from the detailed description of the invention and from the claims.

## DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs.

As used herein, "assessing oxidative stress in a mammal" means obtaining information regarding the production of and/or capacity to produce reactive oxygen species in mammalian cells. Reactive oxygen species (ROS) include free radicals that are highly reactive in biological systems. Examples of ROS include peroxides such as superoxides and hydrogen peroxide; as well as singlet oxygen and peroxynitrite. Such information may provide, for example, insight into the current health of the skin (e.g., to determine whether certain cosmetic or pharmaceutical skin treatments are necessary) as well as provide insight regarding the response of the skin to external trauma or treatment.

According to the invention, oxidative stress of the skin of a mammal is assessed by: a) exposing skin cells of said mammal to an oxidizable moiety; b) exposing said skin cells to an external aggression; and c) assessing a reaction product of said oxidizable moiety. These steps are performed in the order (a) followed by (b) followed by (c). In addition, prior to said assessing step (c), that is, before step (a), or between steps (a) and (b), or between steps (b) and (c), said skin cells are non-invasively removed from said mammal such a manner that the removed skin cells are viable. In this manner, said assessing step (c) is performed on the removed, viable skin cells.

Non-invasive removal of viable skin cells

As used herein, "non-invasive removal of viable skin cells" means the separation and collection of skin cells from the skin of the subject mammal, such that the removal is from the stratum corneum and optionally the epidermis, but not the dermis. In a preferred embodiment, said removal is of skin cells only from the stratum corneum but not the epidermis. "Viable" as used herein means that the skin cells are capable of living, developing, or germinating, as well as metabolic activity, such as enzymatic reactions, under favorable conditions. Skin cells that have been lysed through, for example, exposure to aggressive solvents are not viable.

In one embodiment of the invention, the non-invasive removal comprises surface stripping, i.e., viable skin cells are stripped off the outer surface of the skin of the subject. In one particularly suitable embodiment, the non-invasive removal of skin cells comprises contacting a skin surface of said mammal with an adhesive, wherein said adhesive is attached to a substrate, and forcibly removing said adhesive from said skin surface, thereby causing said skin cells to separate from said skin surface and associate with said adhesive. Such a method is commonly referred to as "tape stripping," wherein a flexible substrate having adhesive bonded thereto is placed into contact with the skin to be removed. Pulling the tape away from the skin removes skin cells from the stratum corneum. These skin cells can then be isolated from the tape.

Examples of suitable tapes that may be used include and D-SQUAME, commercially available from CuDerm Corporation of Dallas, Texas.

Exposing skin cells to an oxidizable moiety

Skin cells, whether on the skin or removed from the skin, are first exposed an oxidizable moiety. As used herein, "moiety" means a chemical compound, element, radical, or ion, or portion thereof. "Oxidizable moiety" means a moiety capable of undergoing a reaction with a reactive oxygen species. The oxidizable moiety is preferably selected such that the reaction with available ROS readily occurs and that ROS react preferentially with the oxidizable moiety as opposed to other cellular components.

In one desirable embodiment, exposure of the skin cells to an oxidizable moiety causes penetration of the skin cells by the oxidizable moiety. As used herein, "penetration" means that the oxidizable moiety moves through the outer membranes of the skin cells and into their interiors. Movement of the oxidizable moiety into the skin cells may be, for example, by passive diffusion or osmosis.

In another embodiment, exposure of the skin cells to an oxidizable moiety comprises providing a chemical species that permeates the skin cells and reacts with one or more chemical species within the skin cells (e.g., enzymes) to form an oxidizable moiety.

In another embodiment, exposure of the skin cells to an oxidizable moiety does not require that the oxidizable moiety penetrate the skin cells, but rather that the oxidizable moiety be in fluid communication with the skin cells. For example, the oxidizable moiety may be positioned outside the skin cells such that ROS generated within the skin cells diffuse through or onto the cell membranes and subsequently react with the oxidizable moiety.

Particularly suitable compounds having oxidizable moieties are described below.

Exposure to external aggression

Next, the skin cells, whether on the skin or removed from the skin, are exposed to an external aggression. In order to allow the skin cells to undergo a spectrum of realistic biochemical responses, it is critical that the skin cells be viable at this step (either on the skin of a living mammal or removed from the skin but still viable as described above). For example, in a viable skin cell, it is possible that a naturally occurring antioxidant, e.g., glutathione, could be biochemically inactivated, thereby affecting the extent of the reaction that would otherwise occur between the ROS and the oxidizable moiety.

Examples of external aggressions include those that are capable of generating ROS in mammalian skin cells, such as cleansers (e.g., skin and hair cleansers containing surfactants) and make-up; shaving and cutting; and environmental factors such as UV light (e.g., from the sunlight or non-natural sources such as UV lamps and solar simulators), ozone, exhaust such as from combustion, pollution, chlorine and compounds containing chlorine, and cigarette smoke. In one particularly notable embodiment, the external aggression is ultraviolet radiation.

Assessing a reaction product of the oxidizable moiety

In response to exposure of the skin cells to the external aggression, a reaction (e.g., chemical reaction) occurs between the oxidizable moiety and the ROS in the skin cells. One or more reaction products are thereby generated.

The oxidizable moiety may be a reactive group or site on a "host compound." The host compound is preferably selected such that at least one reaction product generated by the reaction between the ROS and the oxidizable moiety can be detected, such as by an electromagnetic signal associated with the reaction product. For example, electromagnetic radiation can be made to interact with such reaction product and an electromagnetic signal obtained therefrom can be detected and preferably quantified.

Accordingly, in one embodiment, assessing the reaction product of the oxidizable moiety comprises measuring an electromagnetic emission signal associated with said reaction product. Examples of suitable electromagnetic emission signals include fluorescence signals and luminescent signals such as a chemiluminescent signal. The reaction product may therefore be a fluorescent reaction product.

Host compounds that are particularly suitable are those commonly referred to luminescent, chemiluminescent, or fluorescent "probes" or "molecular probes." These probes may be selected from a wide variety of compounds known in the art. The probe is desirably selected so as to provide a high level of selectivity and sensitivity to ROS, such as peroxides. One class of suitable probe is often referred to as an "internal probe," i.e., one capable of diffusing into skin cells and reacting with ROS therein. One type of internal probe may be enzymatically altered upon diffusion into the cell, rendering the probe capable of generating an electromagnetic signal such as a fluorescence signal essentially only upon reaction with ROS. In other words, such internal probes generate fluorescent reaction products but are, prior to reaction with ROS, not fluorescent.

Examples of cell permeable or internal probes include those listed in "The Handbook - A Guide to Fluorescent Probes and Labeling Technologies, 10th edition" by Richard P. Haugland, and Michelle T.Z. Spence, Invitrogen Corp. Press 2005, including: 2',7'-dichlorodihydrofluorescein diacetate, 2',7'-dichlorofluorescein, carboxy-2',7'-difluorodihydrofluorescein diacetate, 5-(and 6-)chloromethyl-2',7'-dichlorodihydrofluorescein diacetate, acetyl ester, calcein acetoxymethyl ester, dihydrocalcein acetoxymethyl ester, dihydrorhodamine 123, dihydroethidium, 2,3,4,5,6-pentafluorotetramethyldihydrorosamine, biotinylated glutathione ethyl ester, tetrazolium salts such as MTT and XTT. A particularly suitable internal probe is 5-(and -6) chloromethyl-2'7'-dichlorohydrofluorscein diacetate, acetyl ester (CM-H2DCFDA) commercially available from Invitrogen of Carlsbad, California.

Other suitable probes include so called "external probes," i.e., probes that are not capable of penetrating skin cells, but are still capable of reacting with ROS that diffuse from the skin cells.

External probes that may be suitable for use in the present invention those listed in "The Handbook - A Guide to Fluorescent Probes and Labeling Technologies, 10th edition" By Richard P. Haugland, and Michelle T.Z. Spence, Invitrogen Corp. Press 2005, including: 10-acetyl-3,7-dihydroxyphenoxazine , 3'-(p-aminophenyl) fluorescein, diphenyl-1-pyrenylphosphine , R-phycoerythrin, allophycocyanin, fluorescein-labeled phosphatidylethanolamine, and hexadecanoylaminofluorescein.

According to one embodiment, a solution having a concentration of 1-10 mircromolar host compound is exposed to non-invasively removed, viable skin cells.

Providing an extrinsic composition to said viable skin cells

In one embodiment, an extrinisic composition is provided to the viable skin cells. The extrinsic composition is generally applied in order to determine the effect of the composition or components thereof upon either the generation of ROS within the cells or the ability of the cells to generate ROS. The extrinsic composition may include, for example, and antioxidant or a sunscreen.

Suitable antioxidants include, for example, butylated hydroxytoluene (BHT), butylated hydroxyanisole (BHA), beta carotene, alpha hydroxy acids such as glycolic acid, citric acid, lactic acid, malic acid, mandelic acid, ascorbic acid, alpha-hydroxybutyric acid, alpha-hydroxyisobutyric acid, alpha-hydroxyisocaproic acid, atrrolactic acid, alpha-hydroxyisovaleric acid, ethyl pyruvate, galacturonic acid, glucoheptonic acid, glucoheptono 1,4-lactone, gluconic acid, gluconolactone, glucuronic acid, glucuronolactone, glycolic acid, isopropyl pyruvate, methyl pyruvate, mucic acid, pyruvic acid, saccharic acid, saccaric acid 1,4-lactone, tartaric acid, and tartronic acid; beta hydroxy acids such as beta-hydroxybutyric acid, beta-phenyl-lactic acid, beta-phenylpyruvic acid; polyphenolics; botanical extracts such as green tea, soy products, milk thistle, algae, aloe, angelica, bitter orange, coffee, goldthread, grapefruit, hoellen, honeysuckle, Job's tears, lithospermum, mulberry, peony, puerarua, nice, safflower, and mixtures thereof.

Suitable sunscreens include ultraviolet filters, e.g., inorganic or organic (oil soluble or water soluble) filters known to those skilled in the art and commonly employed to absorb or scatter ultraviolet radiation in personal care products. Non-limiting examples include inorganic sunscreens such as oxides of zinc and titanium; organic ultraviolet filters such as benzylidene camphor, 4-Aminobenzoic acid derivatives, specifically 4-(dimethylamino)benzoic acid-2-ethylhexyl esters, 4-(dimethylamino)benzoic acid-2-octyl esters and 4-(dimethylamino)benzoic acid amylesters; esters of cinnamonic acid, esters of salicylic acid, derivatives of benzophenones and benzoylmethane, esters of benzalmalonic acid; triazine derivatives; propane-1,3-diones; ketotricyclodecane derivatives; 2-Phenylbenzimidazol-5-sulfonic acid; sulfonic acid derivatives ofbenzophenones; sulfonic acid derivatives of 3-benzylidene camphor, derivatives of benzoic acid 2-(4-diethylamino-2-hydroxybenzoyl)-benzoic acid hexylester, among other organic filters known in the art.

In a particular notable embodiment of the invention, the extrinsic composition includes an antioxidant and a sunscreen.

The extrinsic composition may include a carrier such as water and various other ingredients suitable for personal care formulations, e.g., surfactants, emulsifiers, emollients, humectants, pH adjusters, fragrances, etc. It is however, desirable, that the extrinsic composition not include ingredients that would substantially interfere with the ability to assess (e.g., quantitatively) the reaction product of interest. As such, if the method includes quantifying a fluorescence signal of the reaction product between the oxidizable moiety and the ROS, it would be desirable to exclude ingredients that fluoresce at the same wavelength as the reaction product.

In one embodiment, the extrinsic composition is topically applied to a subject's skin prior to said noninvasive removal of said viable skin cells. In another embodiment, the extrinsic composition is provided to the skin cells after the skin cells have been removed from the skin.

Oxidation Protection Factor

In one embodiment, an extrinsic composition is topically applied to the skin before exposure to an external aggression. Preferably, the extrinsic composition reduces the effect of the external aggression. Using the method of the invention, the Oxidation Protection Factor, OPF, of such an extrinsic composition may be measured and evaluated.

For example, in one embodiment the external aggression is UV radiation, the reaction product is a fluorescent reaction product, and an extrinsic composition containing a sunscreen is topically applied to the skin before exposing the skin to the UV radiation. The OPF of the extrinsic composition, X, is determined using the method of the invention and the following equation:

$$OPF = 100 \text{ X } (S_{N,UV} - S_{X,UV}) / (S_{N,UV} - S_{N,NUV})$$

where $S_{N,UV}$ is the fluorescence signal obtained when no extrinsic composition is applied to the skin cells, which are then exposed to UV radiation;

where $S_{N,NUV}$ is the fluorescence signal obtained when no extrinsic composition is applied to the skin cells, which are not exposed to UV; and

where $S_{X,UV}$ is the fluorescence signal obtained when extrinsic composition X is applied to the skin cells, which are then exposed to UV radiation.

In one embodiment, a personal care composition comprising a sunscreen is provided having an OPF of at least about 40%, preferably 45%, more preferably 50%. Such a personal care composition may comprise a carrier and/or other suitable ingredients as described above. In particular, the personal care composition may also comprise an antioxidant.

The following non-limiting examples further illustrate the invention.

Example 1

A series of assessments of oxidative stress in human skin caused by various anti-acne compositions (external aggressions) were conducted as follows.

In each case, to provide a consistent baseline skin surface, a 22 mm long D-SQUAME tape having a surface area of 380 mm$^2$ was applied to the inner volar forearm of a human subject, with even mechanical pressure, and left on the skin for one minute. The tape was removed with forceps and discarded. A second tape was then applied to the same site and the application and removal process above was repeated in order to surface strip skin cells. The second tape was placed in the bottom of a 12-well tissue culture plate containing a saline solution (HBSS: Hanks Blanaced Salt Solution). Four replicates were performed for each subject. These tapes, labeled as Reference 1A: "no product," were evaluated as indicated below.

The above process above was repeated. However, prior to application of the second tape a composition containing 10% by weight of benzoyl peroxide (Clearasil Maximum Strength Acne Treatment Cream, commercially available from Reckitt Benckiser of Slough, UK) was topically applied in a surface concentration of 4 milligrams per square centimeter to another site of the subject's inner volar forearm. These tapes were placed in the collection well as above. A similar process was repeated with the only change being the concentration of benzoyl peroxide (BPO). Reference 1B corresponded to 10% benzoyl peroxide; Reference 1C corresponded to 5% benzoyl peroxide (Oxy Lotion; commercially available from Mentholatum Co., Orchard Park, NY); Reference 1D corresponded to 2.25 % benzoyl peroxide (Acne Response Step 3 Blemish Fighting Lotion commercially available from L'Oreal of Paris, France).

Upon removal from the skin, tape stripped cells were immediately incubated for 30 minutes in a 5 micromolar solution of CM-H2DCFDA, a host compound that is hydrolyzed to DCFH within skin cells and forms a fluorescent reaction product, DCF, upon reaction with ROS.

The tissue culture plate was rinsed to remove excess CM-H2DCFDA. The skin tapes were then exposed to 82 KJ/m$^2$ in solar simulator commercially available from Oriel Corp. of Stratford, CT. Assessments were then made for DCF as follows. Each tape was analyzed exactly one hour following irradiation using a fluorescent probe and read on a commercially available spectrofluorometer plate reader from Molecular Devices of Sunnyvale, California, set at an excitation wavelength of 485nm and an emission (detection) wavelength of 530nm. The results, reported as mean fluorescent intensity (MFI) determined using SOFTMAX software also supplied Molecular Devices of Sunnyvale, California, are shown in Table 1 below.

Table 1

| Reference | External Aggression | Fluorescence signal |
|---|---|---|
| 1A | None | 49.74 |
| 1B | 10% BPO | 71.32 |
| 1C | 5% BPO | 63.89 |
| 1D | 2.55% BPO | 39.95 |

These results surprisingly show that by measuring fluorescence according to the method of the invention, one can differentiate between the levels of ROS produced by exposure to different external aggressions, such as various levels of oxidizing compound topically applied to the skin.

Example 2

The Oxidation Protection Factors, OPF, of two topical compositions containing sunscreens was measured and compared as follows. The measurements were conducted in a manner similar to Example 1. Eleven skin tapes were provided for each sample to be evaluated as well as for two controls (described below). Skin tapes were collected from male and female subjects, Skin Type II-III, age range from 25-55 yrs. The skin tapes were exposed to 110 KJ/m$^2$ in the solar simulator.

No extrinsic composition was applied to comparative References 2A and 2B. The extrinsic composition Coppertone Water Babies Spectra 3 SPF 50, commercially available from Schering-Plough, Kenilworth, NJ, was applied to comparative Reference 2D. The following extrinsic composition was applied to Reference 2C according to the invention:

Extrinsic Composition 2C

| INGREDIENT | PERCENT |
|---|---|
|  |  |
| Water | 46.300 |
| Homosalate | 15.000 |
| Oxybenzone | 6.000 |
| Styrene/Acrylates Copolymer | 5.500 |
| Octisalate | 5.000 |

(continued)

| INGREDIENT | PERCENT |
|---|---|
| Avobenzone | 3.000 |
| Silica | 3.000 |
| Octocrylene | 2.790 |
| Diethylhexyl 2,6 - Napthalate | 2.790 |
| Beeswax | 2.000 |
| Glyceryl Stearate (and) PEG-100 Stearate | 1.700 |
| Dimethicone (and) Trimethylsiloxysilicate | 0.500 |
| Caprylyl Methicone | 1.500 |
| Ethylhexylglycerin | 0.900 |
| Sodium Polyacrylate (And) Ethylhexyl Stearate (And) Trideceth-6 | 1.000 |
| Acrylates/C12-22 Alkylmethacrylate Copolymer (and) propylene glycol (and) water | 1.000 |
| Cetyl Dimethicone | 1.000 |
| Xanthan Gum | 0.300 |
| Disodium EDTA | 0.200 |
| Fragrance | 0.150 |
| Dipotassium Glycyrrhizate | 0.100 |
| Methylisothiazolinone and Polyaminopropyl biguanide | 0.200 |
| BHT | 0.070 |
| TOTAL | 100.00 |

The extrinsic compositions were applied to a polymethylmethacrylate plate at a surface coverage of 50 microliters of product per 5 square centimeter of plate. The extrinsic compositions were allowed to sit under ambient conditions to dry for about five to ten minutes. The plates were placed, composition side down, onto a particular tissue culture plate containing the skin cells, fully covering the cultured cells. The cells for samples 2B-2D, with the PMMA plates thereon were exposed to the UV dose for fifteen minutes. Sample 2A was not exposed to UV. The $OPF_x$ for References 2C and 2D were calculated using the formula:

$$OPF_x = 100 \text{ X } (S_{N,UV} - S_{X,UV}) / (S_{N,UV} - S_{N,NUV})$$

where $S_{N,UV}$ was the fluorescence signal for no extrinsic composition, with UV, in this case, 84.55;

where $S_{N,NUV}$ was the fluorescence signal for no extrinsic composition, with no UV, in this case, 27.99;

where $S_{N,UV}$ was the fluorescence signal for extrinsic composition X, with UV exposure, in this case, either 55.02 or 63.89.

The results are shown in Table 2 below.

Table 2

| Reference | Treatment | Fluorescence signal | Oxidation Protection Factor |
|---|---|---|---|
| 2A | No extrinsic composition, No UV | 27.99 | ----- |
| 2B | No extrinsic composition, UV | 84.55 | ----- |
| 2C | Extrinsic Composition-2C, UV | 55.02 | 52% |
| 2D | Coppertone Water Babies Spectra 3 SPF 50-a, UV | 63.89 | 37% |

The results surprisingly show that methods of the present invention can be used to assess the ability of topical compositions to resist/prevent oxidative stress, in particular, oxidative stress generated by UV-induced ROS. Furthermore, it is possible to provide compositions that have a high degree of protection from oxidative stress, as manifested by a high OPF. In particular it is possible to provide personal care compositions having an OPF of at least about 40%, preferably at least about 45%, more preferably at least about 50%.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

**Claims**

1.  A method of assessing oxidative stress in a mammal comprising:

    a) exposing skin cells of said mammal to an oxidizable moiety;
    b) exposing said skin cells to an external aggression; and
    c) assessing a reaction product of said oxidizable moiety;

    wherein prior to said assessing step (c), said skin cells are non-invasively removed from said mammal such that said removed skin cells are viable.

2.  The method of claim 1, wherein said assessing step (c) comprises measuring an electromagnetic signal associated with said reaction product.

3.  The method of claim 1, wherein said reaction product is a fluorescent reaction product.

4.  The method of any preceding claim, wherein said exposing step (a) causes penetration of said skin cells by said oxidizable moiety.

5.  The method of any preceding claim, further comprising providing an extrinsic composition to said skin cells, wherein said extrinsic composition comprises an antioxidant, a sunscreen, or a combination thereof.

6.  The method of claim 5, wherein said providing step comprises topically applying said extrinsic composition to said skin cells prior to said exposing step (b).

7.  The method of claim 5, wherein said providing step is performed before said non-invasive removal of said skin cells from said mammal.

8.  The method of claim 5, wherein said providing step is performed after said non-invasive removal of said skin cells from said mammal.

9.  The method of any preceding claim, wherein said non-invasive removal comprises surface stripping said skin cells.

10. The method of any preceding claim, wherein said non-invasive removal comprises contacting a skin surface of said mammal with an adhesive, wherein said adhesive is attached to a substrate, said forcibly removing said adhesive from said skin surface, thereby causing said skin cells to separate from said skin surface and associate with said adhesive.

11. The method of any preceding claim, wherein said external aggression generates reactive oxygen species by said skin cells.

12. The method of any preceding claim, wherein said external aggression is selected from the group consisting of ultraviolet radiation, smoke, temperature, pressure, chemicals, humidity, and combinations thereof.

13. The method of any preceding claim, wherein said external aggression is ultraviolet radiation.

14. A personal care composition comprising a sunscreen, wherein said composition has an oxidation protection factor of at least about 40%.

**15.** The personal care composition of claim 14, wherein said composition has an oxidation protection factor of at least about 45%.

**16.** The personal care composition of claim 14, wherein said composition has an oxidation protection factor of at least about 50%.

**17.** The personal care composition of any one of claims 14 to 16, further comprising an antioxidant.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RICHARD P. HAUGLAND ; MICHELLE T.Z. SPENCE.** The Handbook - A Guide to Fluorescent Probes and Labeling Technologies. Invitrogen Corp. Press, 2005